# EUROPEAN PATENT APPLICATION

(11) **EP 1 541 141 A1**
(43) Date of publication of application: **15.06.2005**
(21) Application number: 03791413.2
(22) Date of filing: 29.08.2003
(51) Int. Cl.: A61K 31/198, A61K 9/08, A61K 9/10, A61K 9/14, A61K 9/16, A61K 9/20, A61K 9/48, A61P 1/16, A61K 35/00

(54) **THERAPEUTIC AGENT FOR HEPATIC DISEASE**

(30) Priority: 30.08.2002 JP 2002253227
(71) Applicant: Ajinomoto Co., Inc., Tokyo 104-8315 (JP)
(72) Inventor: SONAKA, Ichiro c/o AJINOMOTO CO., INC., Kawasaki-shi, Kanagawa 210-8681 (JP); FUJITANI, Shoji c/o AJINOMOTO CO., INC., Kawasaki-shi, Kanagawa 210-8681 (JP)
(74) Representative: Nicholls, Kathryn Margaret
(86) International application number: PCT/JP2003/011040
(87) International publication number: WO 2004/019928

(57) **Abstract**

This invention provides a composition for novel pharmaceutical agent capable of treating liver diseases such as hepatitis, liver cirrhosis, liver cancer and the like via maintenance and improvement of albumin level in blood, namely, a therapeutic agent for liver diseases containing isoleucine, leucine, valine and alanine as active ingredients, wherein the mass ratio of isoleucine:leucine:valine is 1:1.9-2.2:1.1-1.3, the mass ratio of the total mass of isoleucine, leucine and valine:alanine is 1:0.05-10 and a daily dose per person contains isoleucine 0.2-30.0 g, leucine 0.2-30.0 g, valine 0.2-30.0 g and alanine0.2-50.0 g.

## Description

### Technical Field

The present invention relates to a pharmaceutical composition for the treatment of liver diseases and a treatment method of liver diseases. Liver diseases include hepatitis, liver cirrhosis, liver cancer and the like.

### Background Art

It is known that, in patients with liver cirrhosis, blood Fischer ratio [branched chain amino acid mol (isoleucine + leucine + valine)/aromatic amino acid mol (phenylalanine + tyrosine)] and serum albumin concentration decrease, the serum albumin concentration and Fischer ratio show a positive correlation [Japan Medical Journal (1983) 3101:3], and lower serum albumin concentration is associated with aggravation of disease state [JJPEN(1995)17:208].

Branched chain amino acid preparations are pharmaceutical agents developed for the purpose of correcting Fischer ratio, increasing serum albumin concentration and improving various conditions of liver cirrhosis, by orally supplementing three kinds of branched chain amino acids of isoleucine, leucine and valine at an appropriate ratio.

It is known that branched chain amino acid can be used as an energy substrate even under conditions where carbohydrates are not easily utilized as an energy substrate, such as liver failure and the like [Chronic Disease (1993) 4:411]. Therefore, a part of the branched chain amino acid orally supplemented for improving serum albumin concentration in liver cirrhosis is considered to be consumed as an energy substrate, which suggests a possibility that a serum albumin concentration-improving effect does not necessarily result in a sufficient treatment effect.

### Disclosure of the Invention

The present invention aims at providing a novel pharmaceutical composition effective for the treatment of liver diseases. Another object of the present invention is to provide a novel method of treating liver diseases.

As a result of the intensive studies conducted by the present inventors in an attempt to solve the above-mentioned problems, they have found that a composition containing four kinds of amino acids of isoleucine, leucine, valine and alanine as active ingredients has a Fischer ratio-increasing effect, based on which that the treatment of hepatitis, liver cirrhosis, liver cancer and the like can be performed via maintenance and improvement of albumin level in blood, which resulted in the completion of the present invention. The present invention encompasses the following items.
(1) A therapeutic agent for liver diseases, which comprises isoleucine, leucine, valine and alanine as active ingredients.
(2) The therapeutic agent of the above-mentioned (1), wherein a mass ratio of the total mass of isoleucine, leucine and valine:alanine is 1:0.05-10.
(3) The therapeutic agent of the above-mentioned (1) or (2), wherein the mass ratio of isoleucine:leucine:valine is 1:1.9-2.2:1.1-1.3.
(4) The therapeutic agent of any of the above-mentioned (1)-(3), wherein the dose of the active ingredients per day is 1.0 g - 50.0 g.
(5) The therapeutic agent of any of the above-mentioned (1)-(4), which comprises at least one kind of preparation selected from tablet, granule, powder, capsule and injection, comprising isoleucine, leucine, valine and alanine individually or any combination thereof.
(6) The therapeutic agent of any of the above-mentioned (1)-(5), which comprises all of isoleucine, leucine, valine and alanine in one kind of preparation selected from tablet, granule, powder, capsule and injection.
(7) The therapeutic agent of any of the above-mentioned (1)-(5), which comprises a preparation comprising isoleucine, leucine and valine and a preparation comprising alanine in combination.
(8) The therapeutic agent of any of the above-mentioned (1)-(7), wherein the liver disease is any of hepatitis, liver cirrhosis and liver cancer.
(9) A pharmaceutical composition for the correction of a Fischer ratio, which comprises isoleucine, leucine, valine and alanine as active ingredients.
(10) An enhancer of an effect of a branched chain amino acid preparation to correct a Fischer ratio, which comprises alanine as an active ingredient, wherein the branched chain amino acid preparation comprises isoleucine, leucine and valine as active ingredients.
(11) The enhancer of the above-mentioned (10), which is administered such that a ratio of the total daily dose of isoleucine, leucine and valine in the branched chain amino acid preparation:daily dose of alanine in the enhancer becomes 1:0.05-10.
(12) The enhancer of the above-mentioned (10) or (11), wherein the mass ratio of isoleucine:leucine:valine is 1:1.9-2.2:1.1-1.3 in the branched chain amino acid preparation .
(13) A method of treating a liver disease, which comprises administering effective amounts of isoleucine, leucine, valine and alanine as active ingredients to a patient.
(14) The treatment method of the above-mentioned (13), wherein a ratio of the total daily dose of isoleucine, leucine and valine:daily dose of alanine is 1:0.05-10.
(15) The treatment method of the above-mentioned (13) or (14), wherein the ratio of single dose of isoleucine:single dose of leucine:single dose of valine is 1:1.9-2.2:1.1-1.3.
(16) The treatment method of any of the above-mentioned (13)-(15), wherein a daily dose of the active ingredients is 1.0 g-50.0 g.
(17) The treatment method of any of the above-mentioned (13)-(16), which comprises administering isoleucine, leucine, valine and alanine individually or in any combination thereof.
(18) The treatment method of the above-mentioned (17), which comprises simultaneously administering isoleucine, leucine, valine and alanine.
(19) The treatment method of any of the above-mentioned (13)-(18), wherein the liver disease is any of hepatitis, liver cirrhosis and liver cancer.
(20) A method of enhancing an effect of correcting a Fischer ratio that a branched chain amino acid preparation has, which comprises administering an effective amount of alanine as an active ingredient to a patient, wherein the branched chain amino acid preparation comprises isoleucine, leucine and valine as active ingredients.
(21) The method of the above-mentioned (20), which comprises administering such that a ratio of the total daily dose of isoleucine, leucine and valine in the branched chain amino acid preparation:daily dose of alanine becomes 1:0.05-10.
(22) The method of the above-mentioned (20) or (21), wherein the mass ratio of isoleucine:leucine:valine is 1:1.9-2.2:1.1-1.3 in the branched chain amino acid preparation.
(23) Use of isoleucine, leucine, valine and/or alanine for the production of a therapeutic agent for liver diseases, which comprises isoleucine, leucine, valine and alanine as active ingredients.
(24) The use of the above-mentioned (23), wherein the therapeutic agent for liver diseases, which comprises isoleucine, leucine, valine and alanine as active ingredients, has a mass ratio of the total mass of isoleucine, leucine and valine:alanine of 1:0.05-10.
(25) The use of the above-mentioned (23) or (24), wherein the therapeutic agent for liver diseases, which comprises isoleucine, leucine, valine and alanine as active ingredients, has a mass ratio of isoleucine:leucine:valine of 1:1.9-2.2:1.1-1.3.
(26) The use of any of the above-mentioned (23)-(25), wherein a daily dose of the active ingredients of the therapeutic agent for liver diseases, which comprises isoleucine, leucine, valine and alanine as active ingredients, is 1.0 g-50.0 g.
(27) The use of any of the above-mentioned (23)-(26), wherein the liver disease is any of hepatitis, liver cirrhosis and liver cancer.
(28) Use of alanine for the production of an enhancer of an effect of correcting a Fischer ratio that a branched chain amino acid preparation has, wherein the branched chain amino acid preparation comprises isoleucine, leucine and valine as active ingredients.
(29) The use of the above-mentioned (28), wherein a ratio of the total mass of isoleucine, leucine and valine in the branched chain amino acid preparation:alanine in the enhancer is 1:0.05-10.
(30) The enhancer of the above-mentioned (28) or (29), wherein a mass ratio of isoleucine:leucine:valine is 1:1.9-2.2:1.1-1.3 in the branched chain amino acid preparation.
(31) A commercial package comprising the therapeutic agent of any of the above-mentioned (1)-(8) and a written matter associated therewith, the written matter stating that the therapeutic agent can or should be used for treating a liver disease.
(32) A commercial package comprising the pharmaceutical composition of the above-mentioned (9) and a written matter associated therewith, the written matter stating that the pharmaceutical composition can or should be used for correcting a Fischer ratio.
(33) A commercial package comprising the enhancer of any of the above-mentioned (10)-(12) and a written matter associated therewith, the written matter stating that the enhancer can or should be used for enhancing the effect of correcting a Fischer ratio that a branched chain amino acid has.

### Brief Description of the Drawings

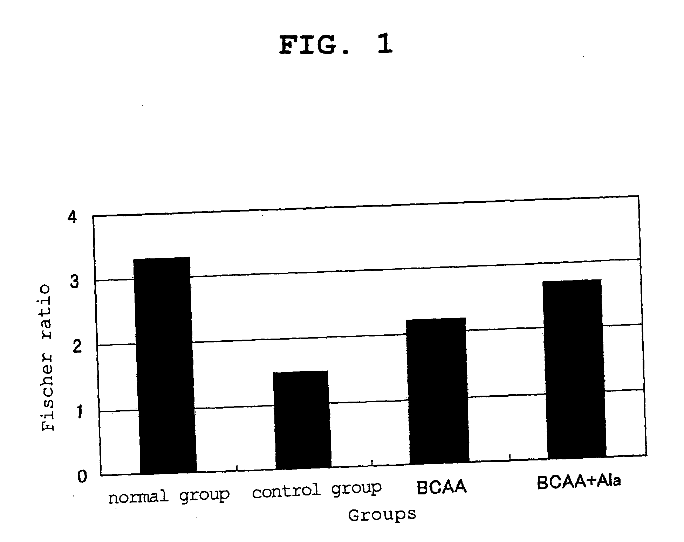

Fig. 1 shows Fischer ratio of plasma at one week after the start of feeding a experimental diet to rats with carbon tetrachloride-induced chronic liver failure.

### Best Mode for Embodying the Invention

The mode of embodiment of the invention is explained in the following.

The pharmaceutical agent of the present invention can be used particularly in the form of a pharmaceutical product (including pharmaceutical composition, drug for liver disease, nutritional supplement etc.), or a form used for food and drink (including health food, dietary supplement etc.).

The therapeutic agent for liver diseases of the present invention can be applied to liver diseases such as hepatitis, liver cirrhosis, liver cancer and the like. Particularly, it is effective for liver diseases associated with reduction of Fischer ratio, and extremely effective for liver diseases associated with reduction of Fischer ratio wherein use of carbohydrates as energy substrate is difficult.

Alanine to be used in the present invention is known to suppress increase of lactic dehydrogenase extracellular overflow due to a D-galactosamine loading in rat primary culture hepatocytes, but other amino acids are known not to have such effect [Hepatology (1996) 24:185]. Furthermore, alanine improves liver failure in D-galactosamine-induced liver failure rat model and raises the level of ATP that becomes lower in liver failure. However, glucose does not provide such effect and alanine is described as being effective as an energy substrate for the liver in liver failure [Hepatology (1996) 24:1211]. In addition, it has been reported that alanine is effective for the treatment of hepatitis such as viral hepatitis, drug-induced hepatitis, fulminant hepatitis and the like (JP-A-5-221858).

Since alanine is used as an energy substrate for the liver in liver failure including liver cirrhosis, and branched chain amino acid is considered to be a main donor of amino group for alanine synthesis, alanine is considered to be synthesized from a part of the branched chain amino acid orally supplemented for increasing Fischer ratio and improving serum albumin concentration in liver cirrhosis, and therefore, a serum albumin concentration-improving effect may not be able to provide a sufficient treatment effect.

Increased doses of branched chain amino acid in an attempt to improve the serum albumin concentration lead to excessive administration of a particular amino acid, thus possibly causing imbalance of amino acids. In the meantime, the effect of improving serum albumin by the combined use of branched chain amino acid and alanine is not clear.

The present invention has first confirmed that the combined use of branched chain amino acid and alanine remarkably improves Fischer ratio, and a serum albumin concentration-improving effect is expected.

The therapeutic agent for liver diseases of the present invention is particularly effective for the treatment, improvement and/or prophylaxis in patients with liver diseases, who showed an insufficient effect on the increase of Fischer ratio and improvement of serum albumin concentration with conventional branched chain amino acid preparations. As shown in the Example to be described below, use of isoleucine, leucine, valine and alanine as active ingredients can afford the object effect. Since the active ingredients are amino acids, moreover, the agent is superior in safety and can be used conveniently even in the form of food or drink (health food etc.).

When the pharmaceutical composition and food of the present invention are used for the treatment or prophylaxis in liver disease patients, they can be administered orally. While the dose varies depending on the condition and age of patients subject to administration and administration method, it generally includes isoleucine in amount of 0.2-30.0 g, leucine 0.2 g-30.0 g, valine 0.2-30.0 g and alanine 0.2-50.0 g for one day. In the case of an ordinary adult, it preferably includes isoleucine in amount of 1.0-10.0 g, leucine 1.0-10.0 g, valine 1.0-10.0 g, alanine 1.0-30.0 g, more preferably isoleucine 2.5-3.0 g, leucine 5.0-6.0 g, valine 3.0-4.0 g, alanine 3.0-20.0 g, for one day.

In addition, the dose of the active ingredients for one day is 1.0 g-50.0 g, more preferably 3.0 g-30.0 g.

A preferable mixing composition in the ratio of the total mass of isoleucine, leucine and valine:mass of alanine is 1:0.05-10, preferably 1:0.2-5.

The mass ratio of isoleucine:leucine:valine is 1:1.9-2.2:1.1-1.3.

An enhancer of the effect of correcting Fischer ratio that the branched chain amino acid has, of the present invention, which contains alanine as an active ingredient, is used in combination with a branched chain amino acid preparation containing isoleucine, leucine and valine as active ingredients. In this case, the daily dose of alanine, which is the active ingredient, is as mentioned above. The enhancer of the present invention is administered such that the total amount of isoleucine, leucine and valine, contained in the branched chain amino acid preparation, and alanine is 1.0 g-50.0 g, preferably 3.0 g-30.0 g, for one day. In this case, the daily dose of isoleucine, leucine and valine is as mentioned above.

In the present invention, the "mass ratio" means a ratio of the mass of each component in the preparation. For example, when respective active ingredients of isoleucine, leucine, valine and alanine are contained in a single preparation, it means a ratio of individual contents, and when each of the active ingredients, or any combination thereof is/are contained in plural preparations, it means a ratio of the mass of each active ingredient contained in each preparation. In the present invention, the "ratio of dose" shows a ratio of a single dose of each active ingredient or a daily dose per one subject of administration (i.e., patient). For example, when each active ingredient of isoleucine, leucine, valine and alanine is contained in a single preparation and administered to a subject of administration, the mass ratio corresponds to the dose ratio. When respective active ingredients are used separately or in any combination thereof in plural preparations, it is a ratio of the total amount of each active ingredient in each preparation administered at one time or in one day.

In the therapeutic agent for liver diseases of the present invention, optical isomers of the aforementioned amino acids to be used as active ingredients are free of any particular limitation, but the use of L-form is desirable.

Isoleucine, leucine, valine and alanine, which are the active ingredients in the present invention, may be contained in a preparation individually or in any combination, or all may be contained in one kind of preparation. For administration after individual processing into preparations, the administration route and the administration dosage form thereof may be the same or different, and the timing of the administration may be simultaneous or separate, which can be appropriately determined based on the kind of pharmaceutical agents to be concurrently used and the effect thereof. For example, a branched chain amino acid preparation containing isoleucine, leucine and valine as active ingredients is available. An embodiment using the branched chain amino acid preparation and alanine in combination is within the scope of the present invention.

In the present invention, an enhancer contains alanine as an active ingredient and potentiates the effect of correcting Fischer ratio that the branched chain amino acid preparation has, by a combined use with the branched chain amino acid preparation containing isoleucine, leucine and valine as active ingredients.

The administration method of the enhancer of the effect of correcting Fischer ratio of the present invention, which contains alanine as an active ingredient, may be any as long as it can be used concurrently with a branched chain amino acid preparation. For example, it may be administered as a branched chain amino acid preparation containing an enhancer, or may be administered by a different administration method or the same administration method, as a preparation permitting individual administration. The timing of the administration is also determined appropriately.

The pharmaceutical composition and food of the present invention can be formulated into a preparation by a conventional method. As the form of the preparation, tablet, granule, powder, capsule, injection and the like can be mentioned. As a carrier for preparation, for example, lactose, glucose, D-mannitol, starch, crystalline cellulose, calcium carbonate, kaolin, gelatin and the like can be mentioned, which may be mixed for use according to the form of the preparation.

These preparations can be administered by any administration method such as oral, injection or topical administration and the like.

### Example

The present invention is explained in more detail by referring to Example in the following. However, the following Example should be considered as an aid to obtain concrete understanding of the present invention, and the scope of the present invention is not at all limited by the following Example.

### Example 1

0.05% aq. sodium phenobarbital was given to 7-week-old male SD rats and 0.5 ml/kg carbon tetrachloride as a 50% olive oil solution was subcutaneously administered to the back twice a week for 25 weeks to prepare rats with chronic liver failure. 0.05% aq. sodium phenobarbital was given, carbon tetrachloride was continuously administered in the same manner as in the preparation of chronic liver failure rats in Example 1, and a experimental diet (see the following Table) was freely given. Blood was taken from subclavian vein one week after the start of the experimental diet, and after deproteinization, amino acid in plasma was analyzed by automatic amino acid analysis and plasma Fischer ratio was determined. The results are shown in Fig. 1.

The Fischer ratio in the Figure shows a branched chain amino acid (isoleucine + leucine + valine)/aromatic amino acid (phenylalanine + tyrosine) ratio, wherein the black bars show normal group, control group, BCAA (branched chain amino acid diet group), and BCAA+Ala (branched chain amino acid+alanine diet group).

| | Normal group | Control group | BCAA | BCAA+Ala |
|---|---|---|---|---|
| (g/100 g experimental diet) | | | | |
| Casein | 21.7 | 21.7 | 20.0 | 20.0 |
| L-isoleucine | 0.0 | 0.0 | 0.6 | 0.6 |
| L-leucine | 0.0 | 0.0 | 1.2 | 1.2 |
| L-valine | 0.0 | 0.0 | 0.7 | 0.7 |
| L-alanine | 0.0 | 0.0 | 0.0 | 2.5 |
| L-methionine | 0.3 | 0.3 | 0.3 | 0.3 |
| Cornstarch | 63.8 | 63.8 | 63.0 | 60.5 |
| Corn oil | 5.0 | 5.0 | 5.0 | 5.0 |
| Choline chloride | 0.2 | 0.2 | 0.2 | 0.2 |
| Cellulose | 4.0 | 4.0 | 4.0 | 4.0 |
| Vitamin mixture* | 1.0 | 1.0 | 1.0 | 1.0 |
| Mineral mixture** | 4.0 | 4.0 | 4.0 | 4.0 |

| | | | | |
|---|---|---|---|---|
| *: Oriental Yeast Co., Ltd. | | | | |
| **: Harper mixture | | | | |

As shown in Fig. 1, BCAA+alanine group showed an improved plasma Fischer ratio as compared to the BCAA group. It is postulated that, in the liver in liver failure, because alanine effectively used as an energy substrate was added, branched chain amino acid taken from the experimental diet was prevented from being utilized as an energy substrate for the liver, and therefore, the branched chain amino acid level in blood increased and the plasma Fischer ratio was improved, which is considered to be not simply an effect of the increased amounts of amino acid and energy substrate.

### Industrial Applicability

From the foregoing description, it is clear that the therapeutic agent for liver diseases, comprising isoleucine, leucine, valine and alanine as active ingredients, which is provided by the present invention, is effective for general liver diseases such as hepatitis, liver cirrhosis, liver cancer and the like. Furthermore, since the therapeutic agent comprises amino acid as an active ingredient, it is highly safe and hardly causes side effects, and therefore, advantageous as a pharmaceutical product.

The present application is based on Patent Application No. 2002-253227 filed in Japan, the contents of which are hereby incorporated by reference.

## Claims

1. A therapeutic agent for liver diseases, which comprises isoleucine, leucine, valine and alanine as active ingredients.

2. The therapeutic agent of claim 1, wherein a mass ratio of the total mass of isoleucine, leucine and valine:alanine is 1:0.05-10.

3. The therapeutic agent of claim 1 or 2, wherein the mass ratio of isoleucine:leucine:valine is 1:1.9-2.2:1.1-1.3.

4. The therapeutic agent of any of claims 1 to 3, wherein the dose of the active ingredients per day is 1.0 g - 50.0 g.

5. The therapeutic agent of any of claims 1 to 4, which comprises at least one kind of preparation selected from tablet, granule, powder, capsule and injection, comprising isoleucine, leucine, valine and alanine individually or any combination thereof.

6. The therapeutic agent of any of claims 1 to 5, which comprises all of isoleucine, leucine, valine and alanine in one kind of preparation selected from tablet, granule, powder, capsule and injection.

7. The therapeutic agent of any of claims 1 to 5, which comprises a preparation comprising isoleucine, leucine and valine and a preparation comprising alanine in combination.

8. The therapeutic agent of any of claims 1 to 7, wherein the liver disease is any of hepatitis, liver cirrhosis and liver cancer.

9. A pharmaceutical composition for the correction of a Fischer ratio, which comprises isoleucine, leucine, valine and alanine as active ingredients.

10. An enhancer of an effect of a branched chain amino acid preparation to correct a Fischer ratio, which comprises alanine as an active ingredient, wherein the branched chain amino acid preparation comprises isoleucine, leucine and valine as active ingredients.

11. The enhancer of claim 10, which is administered such that a ratio of the total daily dose of isoleucine, leucine and valine in the branched chain amino acid preparation:daily dose of alanine in the enhancer becomes 1:0.05-10.

12. The enhancer of claim 10 or 11, wherein the mass ratio of isoleucine:leucine:valine is 1:1.9-2.2:1.1-1.3 in the branched chain amino acid preparation .

13. A method of treating a liver disease, which comprises administering effective amounts of isoleucine, leucine, valine and alanine as active ingredients to a patient.

14. The treatment method of claim 13, wherein a ratio of the total daily dose of isoleucine, leucine and valine:daily dose of alanine is 1:0.05-10.

15. The treatment method of claim 13 or 14, wherein the ratio of single dose of isoleucine:single dose of leucine:single dose of valine is 1:1.9-2.2:1.1-1.3.

16. The treatment method of any of claims 13 to 15, wherein a daily dose of the active ingredients is 1.0 g-50.0 g.

17. The treatment method of any of claims 13 to 16, which comprises administering isoleucine, leucine, valine and alanine individually or in any combination thereof.

18. The treatment method of claim 17, which comprises simultaneously administering isoleucine, leucine, valine and alanine.

19. The treatment method of any of claims 13 to 18, wherein the liver disease is any of hepatitis, liver cirrhosis and liver cancer.

20. A method of enhancing an effect of correcting a Fischer ratio that a branched chain amino acid preparation has, which comprises administering an effective amount of alanine as an active ingredient to a patient, wherein the branched chain amino acid preparation comprises isoleucine, leucine and valine as active ingredients.

21. The method of claim 20, which comprises administering such that a ratio of the total daily dose of isoleucine, leucine and valine in the branched chain amino acid preparation:daily dose of alanine becomes 1:0.05-10.

22. The method of claim 20 or 21, wherein the mass ratio of isoleucine:leucine:valine is 1:1.9-2.2:1.1-1.3 in the branched chain amino acid preparation.

23. Use of isoleucine, leucine, valine and/or alanine for the production of a therapeutic agent for liver diseases, which comprises isoleucine, leucine, valine and alanine as active ingredients.

24. The use of claim 23, wherein the therapeutic agent for liver diseases, which comprises isoleucine, leucine, valine and alanine as active ingredients, has a mass ratio of the total mass of isoleucine, leucine and valine:alanine of 1:0.05-10.

25. The use of claim 23 or 24, wherein the therapeutic agent for liver diseases, which comprises isoleucine, leucine, valine and alanine as active ingredients, has a mass ratio of isoleucine:leucine:valine of 1:1.9-2.2:1.1-1.3.

26. The use of any of claims 23 to 25, wherein a daily dose of the active ingredients of the therapeutic agent for liver diseases, which comprises isoleucine, leucine, valine and alanine as active ingredients, is 1.0 g-50.0 g.

27. The use of any of claims 23 to 26, wherein the liver disease is any of hepatitis, liver cirrhosis and liver cancer.

28. Use of alanine for the production of an enhancer of an effect of correcting a Fischer ratio that a branched chain amino acid preparation has, wherein the branched chain amino acid preparation comprises isoleucine, leucine and valine as active ingredients.

29. The use of claim 28, wherein a ratio of the total mass of isoleucine, leucine and valine in the branched chain amino acid preparation:alanine in the enhancer is 1:0.05-10.

30. The enhancer of claim 28 or 29, wherein a mass ratio of isoleucine:leucine:valine is 1:1.9-2.2:1.1-1.3 in the branched chain amino acid preparation.

31. A commercial package comprising the therapeutic agent of any of claims 1 to 8 and a written matter associated therewith, the written matter stating that the therapeutic agent can or should be used for treating a liver disease.

32. A commercial package comprising the pharmaceutical composition of claim 9 and a written matter associated therewith, the written matter stating that the pharmaceutical composition can or should be used for correcting a Fischer ratio.

33. A commercial package comprising the enhancer of any of claims 10 to 12 and a written matter associated therewith, the written matter stating that the enhancer can or should be used for enhancing the effect of correcting a Fischer ratio that a branched chain amino acid has.
